Europäisches Patentamt

European Patent Office

Office européen des brevets

(19)

(11) Publication number: **0 227 336 B1**

(12) **EUROPEAN PATENT SPECIFICATION**

(45) Date of publication of patent specification: **28.04.93**

(51) Int. Cl.[5]: **C12N 1/20, A01N 63/00, C05F 11/08, A01C 1/06**

(21) Application number: **86309349.8**

(22) Date of filing: **01.12.86**

(54) **Nodulation promoting bacteria and use thereof.**

(30) Priority: **02.12.85 US 803662**

(43) Date of publication of application:
**01.07.87 Bulletin 87/27**

(45) Publication of the grant of the patent:
**28.04.93 Bulletin 93/17**

(84) Designated Contracting States:
**DE FR GB IT**

(56) References cited:
**EP-A- 0 017 565     EP-A- 0 064 720**
**EP-A- 0 083 267     EP-A- 0 256 889**
**WO-A-87/00194       FR-A- 2 193 085**

**BIOLOGICAL ABSTRACTS, vol. 81, 1986, abstract no. 29561, Philadelphia, PA., US; J.W. KLOEPPER et al.: "Measuring the spermosphere colonizing capacity (spermosphere competence) of bacterial inoculants.", & CAN J MICROBIOL 31(10): 926-929. 1985**

(73) Proprietor: **IMPERIAL OIL LIMITED**
**111 St. Clair Avenue West**
**Toronto Ontario, M5W 1K3(CA)**

(72) Inventor: **Polonenko, Daniel R.**
**254 Harvard Drive**
**Port Moody, B.C. V3H 1S8(CA)**
Inventor: **Kloepper, Joseph W.**
**12 Mullen Place**
**Georgetown Ontario, L7G 2R9(CA)**
Inventor: **Scher, Francis M.**
**43 Finchley Crescent**
**Bramalea Ontario, L6T 3P5(CA)**

(74) Representative: **Eyles, Christopher Thomas**
**W.P. THOMPSON & CO. High Holborn House**
**52-54 High Holborn**
**London WC1V 6RY (GB)**

CHEMICAL ABSTRACTS, vol. 95, 1981, page 434, abstract no. 39319x, Columbus, Ohio, US; S.S. DUDEJA et al.: "Effect of rhizobium and phosphomicroorganisms on yield and nutrient uptake in chickpea", & CURR. SCI. 1981, 50(11) 503-5

BIOLOGICAL ABSTRACTS, vol. 78, 1984, abstract no. 46582, Philadelphia, PA, US; H.D. GRIMES et al.: "Influence of pseudomonas putida on modulation of phaseolus vulgaris", & SOIL BIOL BIOCHEM 16(1): 27-30. 1984

CHEMICAL ABSTRACTS, vol. 81, 1984, page 231, abstract no. 60637, Columbus, Ohio, US; A.P. PETROSYAN et al.: "Effect of rhizosphere microorganisms of legumes on the itensity of symbiotic nitrogen fixation. 2. nitrogen-fixing activity of rhizobial bacteria during their simultaneous development with rhizosphere microorganisms.", & VOP MIKROBIOL. 1973, 6,20-30

ANNUAL REVIEW OF PHYTOPATHOLOGY, vol. 12, 1974, pages 181-197; M.E. BROWN: "Seed and Root Bacterizytion"

BIOLOGICAL ABSTRACTS, vol. 84, 1987, abstract no. 61877, Philadelphia, PA, US; D.R. POLONENKO et al.: "Effects of root colonizing bacteria on modulation of soybean roots by bradyrkizobium japonicum" & CAN J MICROBIOL 33(6): 498-503 1987

Current Science 1981, Vol. 50, N 11, pp. 503-505, Soil Biol. Biochem. 1984, Vol. 16, pp. 27-30

**Description**

BACKGROUND OF THE INVENTION

This invention relates to compositions and methods for enhancing crop growth characteristics, and more particularly to bacterial treatments of soils, seeds, crop roots and crop growth environments to improve crop growth and yields.

Certain types of soil bacteria of the family Rhizobiaceae including Rhizobium and Bradyrhizobium - (hereinafter referred to as rhizobia) are capable of entering into a symbiotic relationship with leguminous plants. There appears to be a rather high degree of specificity between plant and bacterial species. Infection and nodulation of legumes by appropriate rhizobial strains can result in significant increases in plant growth and yields. This feature has led to the development of numerous inoculant formulations containing rhizobia in attempts to increase the efficiency with which this symbiosis occurs so that improved crop yields may be realized.

Although the beneficial effects of the rhizobia/legume symbiosis are easily demonstrated under controlled conditions in growth chambers and greenhouses, rhizobial inoculants often fail to stimulate significant improvements in the field (see for example Dunigan et al, 1980, Lou. State. U. Bull #726). The reasons for poor performance of the rhizobial inoculants are different for the two distinct situations in which these products are used, namely, (1) soils with no prior history of the legume crop and concomitant use of inoculant, and (2) soils which contain indigenous rhizobia because they were previously planted with the respective legume and perhaps also treated with appropriate bacterial inoculant for the respective legume.

In the first situation, when soils have no prior history of the particular legume crop to be planted, the seed or soil is treated with an inoculant product that contains appropriate rhizohia strains (Muldoon et al., 1980, Can. J. Plant Sci. 60:399-409; Semu and Hume, 1979, Can. J. Plant Sci. 59:129-137). This is required to ensure satisfactory nodulation of the plants so that sufficient nitrogen will be fixed, which in turn should provide acceptable crop yields. Failure to achieve acceptable nodulation of legumes by rhizobial inoculants is largely due to the failure of the introduced strains to survive in the soil, and to co-exist with the indigenous soil microbial population. Additionally, rhizobia are at a competitive disadvantage to other rhizosphere microorganisms, because strains of rhizobia generally are incapable of "explosive" root colonization and cannot keep pace with growing roots (Reyes and Schmidt, 1981, Plant Soil 61:71-88).

In the second situation, soils previously planted with legumes invariably contain large populations of indigenous rhizobia that can easily out-compete inoculant strains for nodule occupancy (Robert and Schmidt, 1985 Soil. Biol. Biochem. 17:579-580) and can persist in soils for extended periods of time (Nelson et al., 1978 Agron J. 70:517-518). Although nodule mass formed by indigenous rhizobia is similar to that formed by introduced inoculant strains, the indigenous rhizobia are usually less efficient nitrogen-fixers (Reyes and Schmidt, 1981, Plant Soil 61:71-80). This is thought to be a consequence of the need to adapt to ensure survival in the soil environment in the absence of a suitable host plant.

In recent years there have been many attempts to increase the overall efficiency of the host plant/rhizobia interaction, with the aim of increasing crop yield. In general, these attempts have fallen into two broad categories:

i. Increasing the effectiveness of the selected strain of rhizobial strain, and

ii. Increasing the degree to which the host plant is nodulated by the desired strain.

Strategies employed in the first category have included recombinant DNA to improve nitrogen fixing or hydrogen uptake, mutagenesis, strain selection and greater attention to strain/cultivar interaction. To-date, little progress has been reported in making improvements that would be practical at the field level.

There are fewer reports of research designed to increase the degree to which the desired strain nodulates the host plant. This research includes attempts to increase competitiveness of certain strains and/or to decrease the comepetitiveness of indigenous strains. For example, Grimes and Mount (1984, Soil Biol. Biochem. 16:27-30) co-inoculated a strain of Rhizobium phaseoli with Pseudomonas putida to test for ability to control pathogens of beans (Phaseolus vulgaris. S.S. Dudeja et al, (Current Science, June 5, 1981, Vol. 50, No. 11, pages 503 to 505) also described the co-inoculation of Rhizobium sp. with Pseudomonas striata or Aspergillus awamorii or both. An unexpected observation was that the number of nodules was increased under certain conditions. However, plant growth was not affected and yield increases were not obtained. In another study (Fuhrmann and Wollum, 1985, 10th North American Rhiz. Conf. Book, page 959) using an artificial plant growth medium, certain rhizosphere isolates (referred to as pseudomonads) were reported to affect nodulation competition between different strains of the same rhizobia species such that one strain could increase its nodule occupancy at the expense of another rhizobia strain. No increase in overall nodulation was reported.

It is an object of the present invention to provide a novel process for enhancement of crop growth characteristics.

It is a further object of the invention to provide novel bacterial inoculation systems for enhancement of crop growth characteristics.

It is a further object to provide a novel process for identification and selection of microorganisms that will enhance the ability of rhizobial strains to nodulate their target legume hosts.

It is a further object to provide novel bacterial inoculation systems incorporating selected microorganisms with appropriate rhizobial strains, for the enhancement of nodulation and nitrogen fixation on target legume crops.

It is a further object to provide novel bacterial inoculants of selected microorganisms, without additional rhizobial strains, for improvement of nodulation and nitrogen fixation in target legume crops, in soils containing indigenous rhizobial populations.

## SUMMARY OF THE INVENTION

The present invention provides concepts and procedures which enable selection of microorganisms, isolated from soil and root regions, that are aggressive root colonizers and that can substantially improve the nodulation efficiency of strains of rhizobia, regardless of whether the rhizobial strains are indigenous or are provided in a soil or seed inoculant. Thus it defines a novel and advantageous approach to improving the overall efficiency of the rhizobia/host plant interaction.

The selected microorganisms for use in the present invention are defined as nodulation-promoting rhizobacteria, (herein for convenience referred to as NPR). They are characterized by possessing, to a high degree, the properties of chemotactic activity and explosive root colonizing ability on the selected legume. These properties allow the NPR to be highly competent in the legume rhizosphere. In addition, they stimulate nodule mass formation and increase nitrogen fixation.

Thus a unique aspect of this invention is that the NPR microorganisms provided and used herein are selected only from strains which have shown rhizosphere competence as indicated by high chemotactic activity and superior root-colonizing ability. This appears to allow them to achieve more consistent effects by dominating the rhizosphere and root region for the period of time required to establish the nodulation process.

The chemotactic activity of a soil microorganism with respect to a plant root can be assessed on the basis of the response of the microorganism to the respective root exudate, particularly to the amino acids present in the root exudate. Chemotaxis to root exudates, particularly to the amino acids contained in the root exudates, is identified as a trait that enables soil microorganisms successfully to colonize root surfaces. For example, asparagine is a major amino acid constituent of soybean root exudates (Scher et al, Can. J. Microbiol 31:570-574), and can therefore be used to determine the potential of candidate NPR strains, to utilize the soybean root exudate as a substrate and thereby successfully to colonize soybean roots. The test may be conducted using an agar plate containing the chosen amino acid in soft agar, and inoculating it at the centre with a pure culture of the candidate strain. The plate is incubated at 30°C for 24 hours. The diameter of the microbial colony formed during that time is a measure of chemotactic activity (chemotaxis) to the chosen amino acid. The term "high chemotactic activity" as used herein defines strains which develop at least a 20mm diameter microbial colony within 24 hours under the foregoing test.

Explosive root colonizing ability is the second characterizing feature of an NPR used in the present invention. This capacity aggressively to colonize root surfaces enables the NPR strains to influence the rhizosphere in a manner such that nodulation by rhizobia is enhanced. Root-colonizing ability may be determined by inoculating seeds of the legume plant under study with the microbial strain, and planting the inoculated seeds in unsterilized field soil/perlite (1:1) mixtures, in plastic tubes of size 4 x 23cm, one seed per tube, the tubes being half-filled with the soil mixture. The tubes are then heat-sealed to create an enclosed micro-environment, and incubated for 7 days at room temperature under typical day/night conditions. Then the seedlings are harvested by cutting open the plastic tubes and removing the seedlings. Excess soil is shaken from the roots, after which the fresh weight of the roots is determined. The roots are then placed into 9 ml of 0.1M magnesium sulphate solution and vigorously mixed A sample of the resulting suspension is plated onto Pseudomonas agar F (PAE, Difco Prod.), the plate incubated for 48 hours at 30°C after which the number of bacteria on the plate is determined. The results are expressed as the base ten logarithm of the number of colony-forming units thus determined, per gram of root (LOG cfu/g).

The term "explosive root colonizing ability" as used herein refers to microorganisms which with respect to the chosen plant produce a value of at least LOG 2.5 cfu/g of root, when inoculated onto seeds in the foregoing test.

Accordingly, a protocol is used to select and define rhizosphere competent NRR useful in the present invention, thus:

Test 1 - chemotactic activity - must produce at least a 20 mm diameter microbial colony when tested as above, so as to be classed as having high chemotactic activity;

Test 2 - root colonizing ability - must produce at least LOG 2.5 cfu/g gram of root of the chosen plant when tested as above, so as to be classed as explosive root colonizers.

In addition, preferred NPR's used in the present invention will effect stimulation of nodule mass formation and increased nitrogen fixation.

According to one aspect of the invention, there are provided cultures comprising at least one rhizobacterial strain that promotes nodulation of roots of a leguminous and a Bradyrhizobium strain. The nodulation-promoting rhizobacteria are characterized by high chemotactic activity and explosive root-colonizing ability with respect to the selected plant. It is preferred that in the mixed cultures comprising the NPR and the Bradyrhizobium strain there is present an axenic culture of the NPR strains. By "axenic" it is meant that such a culture comprises only clones of the parent organism together with a minor proportion of mutant progeny, as expected. The cultures described herein are devoid of any component which acts deleteriously to inhibit the nodulation-promoting function of the bacteria forming the culture, although innocuous bacteria or chemical agents may be present in minor amounts.

In another aspect of the present invention, there is provided an inoculant composition for treating seeds or soil comprising a nodulation-promoting bacteria, optionally in admixture with an agriculturally acceptable carrier, said nodulation-promoting bacteria being characterized by high chemotactic activity and explosive root-colonizing ability with respect to the selected plant.

From a further aspect, the present invention provides a process for enhancing the nodulation of leguminous plant roots caused by a rhizobial strain which comprises applying to the root-growth environment of the leguminous plant an effective amount of a nodulation promoting rhizobacteria in admixture with a Bradyrhizobium strain.

The term "root-growth environment" is used for convenience herein to define the region in which the root will germinate in the case of seed or the region in which the root is growing in the case of seedlings or maturing plants. Root-growth environments therefore include the seed coat, the root surface or the soil surrounding the seed coat or plant root.

The term "effective amount" is used herein to define that population of bacteria which, in the case of NPR, will enhance the nodulation efficiency of a rhizobial strain present in the root growth environment of the legume. In the case of rhizobia, "effective amount" defines that population of rhizobia which, when in the presence of a given NPR population, will be required to achieve a nodulation efficiency, in terms of nodule number or nodule mass or both, which is suitable from an agricultural viewpoint.

Whilst it is not intended that this invention should be limited by specific mechanisms of action by the NPR in root regions or by other theoretical considerations, the following suggested modes of action are offered for a further and better understanding of the invention.

As previously noted, soils containing no natural rhizobial population for the plants to be grown therein are normally planted with seeds treated with an appropriate rhizobial strain, which must be able to survive in the soil. The successful establishment of these strains in the rhizosphere of the target plant must, therefore, be largely dependent on the presence of a compatible microbial population in the root region. All NPR strains are highly efficient root colonizers but are normally present in soils and root regions at concentrations too low to exert any influence on the activity of added rhizobia. However, when high concentrations of NPR are co-inoculated with rhizobia in the root-growth environment, the NPR strains will tend to dominate the rhizosphere population for a period of time in a manner such that a root environment is created for the infectivity of the rhizobia or for the enhanced susceptibility of the host legume to infection by the given rhizobial strain with the result of markedly improving the nodulation efficiency. This rhizosphere dominance is closely related to chemotactic ability to root exudates and to root colonizing ability. Thus, effective NPR candidates are microorganisms which have demonstrated a high degree of competence in these areas.

Soils previously planted with the leguminous crop contain large populations of indigenous rhizobia. As noted above, certain rhizosphere isolates (pseudomonads) have been used under greenhouse conditions to affect nodulation competition between different strains of B. japonicum such that one strain would increase its nodule occupancy at the expense of another. NPR are quite different and distinct from these strains because, instead of interfering with the ability of the rhizobia bacteria to nodulate their target host, NPR promote nodulation efficiency by both "inoculant" rhizobial strains and those referred to as "indigenous" rhizobial strains. Accordingly, inoculant preparations containing only NPR can be formulated for application in areas that contain large populations of indigenous rhizobia. The NPR will apparently dominate the rhizosphere by "explosive" root colonization, with the result that nodulation by any rhizobial strain is enhanced.

## DESCRIPTION OF THE PREFERRED EMBODIMENTS

The preferred leguminous plant for use in the present invention is soybeans. The rhizobia species useful in nodulation of soybeans is therefore Bradyrhizobium japonicum, as represented by way of example by the known strains Bradyrhizobium japonicum 110, 122, 61A101C, and 122SR. As reported in the specific examples below, at least twenty-six specific bacterial strains have been isolated which are effective as NPR in conjunction with Bradyrhizobium japonicum 110 or 122 in promoting nodulation of soybeans. However, the same NPR strains are similarly effective for all Bradyrhizobium japonicum strains tested, both added and indigenous.

Indeed, the NPR bacteria of the present invention may be applied on root-growth environments of other legumes in order to enhance growth thereof. The NPR are coinoculated, if necessary with the rhizobial strain known to infect the selected legume, e.g. Rhizobium trifolii in the case of white clover, in that embodiment.

While the NPR strains of the present invention are (at least for the most part) naturally-occurring bacterial strains, isolated from soil and root environments of the growing plant, under natural conditions they are present in extremely small amounts. In the present invention, they are used in concentrations in the environment of the plant many times greater than the concentrations found indigenously, so that they may effectively enhance the ability of Bradyrhizobium japonicum to nodulate the host plant.

For example, inoculant compositions which result in a population of from log 2 to log 7 cells on the seed are suitable although cell populations which range from log 4 - log 6 on the inoculated seed are more preferred.

The NPR are located in and isolated from soil samples in which the legume has been grown or from the legume root by standard isolation and screening techniques. One of their characterizing features is that they are root-colonizing bacteria with respect to the selected legume. The corresponding alteration in the composition of the indigenous rhizosphere populations by use of the NPR according to the present invention enhances in some way the nodulation effects of the Bradyrhizobium japonicum, to the benefit of nitrogen fixation by the nodules and consequent growth of the plant and ultimately, crop yields.

The present invention may be put to practical use in agriculture in a number of different ways. In one preferred manner, a dual inoculant system of an NPR and an appropriate Bradyrhizobium japonicum strain is used, for treatment of seeds or application to the soil of the growing environment. The population of Bradyrhizobium on the seed, when applied together with the NPR bacteria as a dual inoculant, is appropriately within the same cell population range as is suitable for NPR bacteria in general i.e. from log 2 - log 7, more preferably from log 4 - log 6. The presence of the Bradyrhizobium japonicum as a constitutent of the dual inoculant ensures that the soil will contain an effective Bradyrhizobium strain to promote the nodulation and growth of the soybean plants. Its presence in the combined inoculant may be superfluous in cases where seeds are to be planted in a soil which has previously grown soybeans, since such soils will contain residual indigenous Bradyrhizobium japonicum strains. Nevertheless in such situations, the presence of Bradyrhizobium japonicum such as Bradyrhizobium japonicum 110 in the dual inoculant system of the present invention safeguards against the possibility that insufficient Bradyrhizobium is present, or that the indigenous Bradyrhizobium strains present in the soil are less effective in nodulating ability. When a soil is to be used in a subsequent season for the growth of soybeans, it is unlikely that B. japonicum inoculation is necessary in the second season, but is nevertheless a safeguard. The NPR forming the other constituent of the co-inoculant composition should be administered every year, since it is unlikely that sufficient quantity of NPR will survive from season to season, to provide the desired nodulation-promoting effect.

Prepared compositions containing the two bacteria may be prepared ahead of time and stored, under controlled conditions, and applied to the seeds or the soil at the time of planting. The seeds may be coated or otherwise treated with the dual inoculant system by dipping of the seeds into a liquid composition containing them, spraying the seeds with such liquid composition, or coating seeds with a slurry comprising bacteria and a nutrient or carrier medium such as oils, etc. When the dual inoculant system is to be applied to the soil, it is suitably mixed with an agriculturally acceptable solid base such as peat. Seeds treated with the dual inoculant system may be stored for reasonable periods of time, prior to planting, and the beneficial effects still obtained. It is not necessary that the seeds or the growing environment be treated simultaneously with the B. japonicum and the NPR. They can be applied separately, and in either order. It is merely necessary that both strains be present in reasonable, effective quantities in the growing environment, at the time of nodulation of the soybean plant roots (normally within 14-21 days after planting). When applying the inoculant on the seed or to the root environment, it should be borne in mind that the bacterial concentration in the inoculant should be slightly greater than the cell population desired to be introduced, to account for mortality of bacteria during processing and storage, when necessary. Since mortality will usually account for a loss of about log 2 of the initial bacterial concentration in the inoculant, the inoculant should contain a concentration of from log 4 to log 9 of the desired bacteria, in order to result in a concentration of from log 2 to log 7 of the applied bacteria in the inoculant at the time of use. More preferably, the inoculant composition comprises a bacterial concentration of from log 6 to log 8 so that the preferred bacterial cell population on the seed or at the root is within the preferred log 4 - log 6 cell range.

The specific preferred NPR strains for use in the present invention were isolated by the procedures described in Examples 1-3 below, and are reported, together with their binomial classification in Table 3 and Example 3 below. Viable typical, representative cultures thereof have been deposited in the American Type Culture Collection and are kept therein under conditions affording permanence to the viable deposits. ATCC reference numbers for the deposited cultures are also given in Table 3 hereafter. It will be appreciated that the present invention encompasses progeny of the deposited material, including clones and sub-clones of the organisms so deposited. In addition, cultures of all specific NPR referred to herein are currently maintained in a permanently viable state at the laboratories of Allelix Inc., 6850 Goreway Drive, Mississauga, Ontario, Canada. They will be maintained in this condition throughout the pendency of this application and, during that time, will be made available by Allelix Inc. to those authorized under relevant patent laws upon request. Persons seeking samples of these cultures are requested to identify the strain by the strain designation code given in Table 3.

The invention will be further described with reference to the following specific, non-limitative examples.

EXAMPLE 1

The soil microorganisms used in these examples were isolated from various diverse soils and root regions. To isolate microorganisms from soil, the field soil was serially diluted in 0.1 M magnesium sulphate solution. 0.1 ml volumes were plated onto plates of Pseudomonas agar F (PAF) and were incubated at 30°C for 24 hours. Single colonies were picked from the plates and were streaked onto fresh PAF agar to obtain single pure colonies.

To isolate soil microorganisms from plant roots, the root samples were removed from the soil, and any adhering soil removed by gentle washing in sterile distilled water. 1-cm long sections of root were placed onto asparagine soft agar (containing 1g asparagine and 2g Bacto-agar in 1.0 l of distilled water). The samples were incubated at 20°C for 24 hours. At the end of the incubation period, a "ring" formed around the root. This "ring" was composed of bacterial strains that showed exceptional chemotactic response to asparagine, a major amino acid component of soybean root exudate. A loopful of bacteria was aseptically transferred from the outer edge of the "ring" to fresh PAF agar in order to purify the bacteria by streaking for single colony recovery. The resulting individual colonies were removed, transferred to PAF agar slants and were termed candidate NPR.

Following this enrichment procedure, the recovered bacterial strains were selected further on asparagine soft agar in the manner described in Example 2.

EXAMPLE 2: CONFIRMATION OF CHEMOTAXIS TO ASPARAGINE

Chemotaxis to root exudates, particularly to the amino acids constituents thereof, is one of the traits that enables soil microorganisms successfully to colonize root surfaces (Scher et al., 1985, Can. J. Microbiol. 31: 570-574).

Asparagine is a major amino acid constituent of soybean root exudates (Scher et al., 1985, Can. J. Microbiol. 31: 570-574) and therefore, was used to confirm the potential of candidate NPR strains selected as described in Example 1 to colonize soybean roots.

The centre of an agar plate containing asparagine soft agar was inoculated with a pure culture of a candidate NPR strain and was incubated at 30°C. The extent of chemotaxis was determined by measuring the diameter of the microbial colony formed after 24 and 36 hours of incubation. The chemotactic activity of some strains later confirmed as NPR strains by the method described in Example 3 is given in Table 1.

TABLE 1

| CHEMOTAXIS OF TYPICAL NPR STRAINS TO ASPARAGINE | | |
|---|---|---|
| Strain | DIAMETER OF MICROBIAL COLONY (mm) | |
| | 24h | 36h |
| G2-8 | 30 | 65 |
| G2-9 | 22 | 50 |
| G8-5 | 20 | 48 |
| G11-32 | 33 | 71 |
| G11-57 | 24 | 57 |

The results indicate that NPR strains exhibit a minimum chemotactic response on asparagine soft agar, of 20mm in 24 h when incubated at 30°C.

EXAMPLE 3 - SCREENING MICROBIAL STRAINS ISOLATED FROM SOILS AND ROOT REGIONS FOR NODULATION-PROMOTING ACTIVITY

In order to assay for ability of candidate NPR strains to promote rhizobial nodulation, candidate NPR strains obtained as described in Example 1 were grown initially in tryptic soy broth (TSB) at 30°C for 24 hours. The cells were harvested by a process involving centrifugation, with discard of the supernatant liquid, washing of the resultant microbial pellet twice in 0.1 M magnesium sulphate solution and resuspending to a final concentration of LOG 8 cfu/ml. Bradyrhizobium japonicum strain 110 was grown in yeast extract mannitol (YEM) broth for 5 days. The cells were harvested as described above except that the B. japonicum was prepared to a final concentration of LOG 5 cfu/ml.

Soybean seeds (cv. 'Maple Arrow' ) were surface sterilized in 2% calcium hypochlorite as described by Kosslak and Bohlool (1984, Plant Physiol. 75:125-130) and then incubated in suspensions containing B. japonicum 110 (LOG 5 cfu/ml) plus 1 NPR strain (LOG 8 cfu/ml). The inoculated seeds were planted in 7-cm pots (1 seed per pot; 6 replicates per treatment) containing an artificial plant growth medium, Promix-C, Plant Products Ltd. Brampton, Ontario and were grown in a greenhouse for five weeks. Controls consisted of seeds inoculated with only B. japonicum 110. The number and weight of nodules per root system were determined at the conclusion of each study. The effects of some candidate NPR strains later confirmed to be NPR on nodule number and nodule mass (i.e. weight of nodules) formed by B. japonicum are given in Table 2 below.

## TABLE 2

### EFFECT OF NPR ON NODULATION BY B. JAPONICUM

| NPR Strain | % increase compared to B. j. control | |
|---|---|---|
| | Number of nodules * | Weight of nodules * |
| 86-64 | 130 | 200 |
| G2-8 | 62 | 233 |
| G2-9 | 82 | 600 |
| G8-4 | 99 | 225 |
| G8-5 | 67 | 550 |
| G8-6A | 106 | 450 |
| G8-32 | 127 | 475 |
| G11-32 | 53 | 143 |
| G11-47 | 14 | 147 |
| G11-57 | 28 | 133 |
| G2-26 | 76 | 400 |
| G11-28 | 33 | 107 |
| G11-71 | 43 | 79 |
| G12-22 | 48 | 47 |
| G14-21 | 40 | 283 |
| 1-102 | (not determined) | 67 |

*Mean of 6 replicates per treatment

Those strains enriched and screened on asparagine soft agar and exhibiting the ability to enhance nodulation efficiency are termed nodulation-promoting rhizobacteria (NPR).

The strains isolated in this manner were identified and characterized by their Gram-stain reaction, growth on various carbohydrates, ability to form pigments, motility, spore formation, and by their responses to biochemical tests included in API-20E strips (API Laboratory Products Ltd.).

Typical nodulation-promoting rhizobacteria thus obtained are listed in Table 3:

TABLE 3

| IDENTIFICATION OF TYPICAL NODULATON-PROMOTING RHIZOBACTERIA | | |
|---|---|---|
| Strain Number | Strain Type | ATCC Reference Number |
| 86-64 | Bacillus megaterium | 53289 |
| G2-8 | Pseudomonas putida | 53288 |
| G2-9 | Pseudomonas fluorescens | |
| G2-26 | Pseudomonas putida | |
| G8-4 | Pseudomonas putida | |
| G8-5 | Pseudomonas putida | 53286 |
| G8-6A | Serratia liquefaciens | |
| G8-32 | Pseudomonas putida | |
| G11-28 | Pseudomonas fluorescens | |
| G11-47 | Pseudomonas putida | |
| G11-57 | Pseudomonas putida | |
| G11-71 | Pseudomonas putida | |
| G12-22 | Pseudomonas fluorescens | |
| G14-21 | Pseudomonas putida | |
| G11-32 | Pseudomonas putida | |
| 1-102 | Serratia proteamaculans ss.quinovora | 53287 |
| 2-68 | Serratia liquefaciens | |
| G20-18 | Pseudomonas fluorescens | 53449 |
| 2-16 | Serratia liquefaciens | |
| G24-14 | Pseudomonas putida | |
| 2-114 | Serratia fonticola | 53450 |
| 55-14 | Pseudomonas putida | |
| 17-29 | Pseudomonas putida | |
| 1-226 | Pseudomonas fluorescens | |
| 17-76 | Pseudomonas putida | |
| 17-34 | Pseudomonas fluorescens | |

## EXAMPLE 4 - ROOT COLONIZATION BY NPR

Variants of NPR strains that exhibited an inherent resistance to 100 mg per ml rifampicin (Rif) were selected as outlined by Schwinghammer and Dudman (1980, IN: Methods for Evaluating Biological Nitrogen Fixation, E.J. Bergerson (Ed.), John Wiley and Sons Ltd., pp 337-365). The Rif-resistant mutants of NPR were grown and harvested as described previously. Soybean seeds were sterilized and inoculated with the NPR as described previously.

A heat-sealed plastic tube (polypropylene, 4x23 cm) was half-filled with unsterilized field soil:perlite mix (1:1, V:V). The inoculated seeds were planted in the tubes (1 seed/tube) and the tubes where then heat-sealed to create an enclosed micro-environment.

After a 7-day incubation at room temperature under typical day/night conditions, the soybean seedlings were harvested by cutting open the plastic tubes and removing the seedlings. Excess soil was shaken from the roots after which the fresh weight of the roots was determined.

The roots were then placed into 9 ml of 0.1 M magnesium sulphate solution and vigorously mixed. Samples of the resulting suspensions were plated onto PAF agar which contained 100 ug/ml Rif. The plates were incubated for 24-48 hours at 30°C after which the numbers of bacteria per plate were determined.

The root-colonizing potential of typical NPR as determined above is given in Table 4.

TABLE 4

| ROOT COLONIZATION BY NPR | |
|---|---|
| Strain | LOG cfu per g root |
| 86-64 | 3.7 |
| G2-8 | 2.8 |
| G2-9 | 3.7 |
| G8-6A | 5.4 |
| G8-32 | 3.8 |

The capacity aggressively to colonize root surfaces enables the NPR strains to influence the rhizosphere in a manner such that nodulation by a rhizobia is enhanced. A value of LOG 2.5 cfu/g root is used here to indicate root colonization.

The results in Table 4 confirm that the NPR strains are efficient root colonizers, generally approaching or surpassing the values obtained in previous studies (Scher et al, 1984, Can. J. Microbial. 31: 151-157).

EXAMPLE 5 - EFFECTS OF NPR ON NODULATION IN FIELD SOIL

Pure cultures of NPR were prepared to a final concentration of LOG 8 cfu/ml, and B. japonicum 110 was prepared to a final concentration of LOG 5 cfu/ml as previously described. 'Maple arrow' soybean seeds were surface-sterilized and inoculated with the mixture as previously described, individual treatments consisting of B. japonicum 110 plus a single NPR strain. Controls were also prepared, the first control seeds being uninoculated and the second control seeds being inoculated with B. japonicum 110 only.

The number and weight of nodules per root system were determined at weekly intervals for 35 days after planting the seeds so prepared in field soils, and maintained at 25°C with a day/night cycle of 14/10 hours, which is representative of field conditions. The treatment means were compared using analysis of variance (ANOVA) followed by the least-significant-difference test (LSD). Table 5 below shows the results on the numbers of nodules per root system so obtained, and Table 6 shows the results for weight of nodules per root system so obtained.

## TABLE 5

EFFECT OF NODULATION-PROMOTING RHIZOBACTERIA ON
NODULATION OF SOYBEANS BY B. JAPONICUM 110
(number of nodules/root system)[a]

| Treatment | Age of Plants (Days) | | | |
|---|---|---|---|---|
| | 14 | 21 | 28 | 35 |
| Uninoculated control | 0 | 0 | 0 | 0 |
| B.j. 110 control | 8.4 | 10.6 | 14.2 | 19.7 |
| 110 + 86-64 | 9.2 | 11.0 | 27.8* | 19.0 |
| 110 + G2-8 | 9.2 | 16.8 | 17.2 | 24.3 |
| 110 + G2-9 | 10.6 | 14.6 | 14.0 | 18.0 |

| Treatment | | | | |
|---|---|---|---|---|
| 110 + G8-5 | 12.2 | 14.0 | 18.8 | 24.0 |
| 110 + G8-6A | 9.4 | 18.2* | 20.4 | 24.0 |
| 110 + G8-32 | 16.6 | 13.0 | 21.2 | 24.8 |
| 110 + G11-32 | 13.2 | 18.2* | 26.8* | 18.8 |
| 110 + G11-57 | 13.6 | 22.8* | 28.2* | 21.5 |
| LSD | 8.6 | 7.5 | 8.6 | 11.6 |

a      means of 5 plants/treatment.

*      Significantly different from the B. japonicum control (P=0.05).

## TABLE 6

EFFECT OF NODULATION-PROMOTING RHIZOBACTERIA ON DRY
WEIGHTS OF NODULES FORMED BY SOYBEANS INFECTED WITH
B. JAPONICUM 110 (DRY WT(G) NODULE/ROOT SYSTEM)[a]

| | Age of Plants (Days) | | | |
|---|---|---|---|---|
| Treatment | 14 | 21 | 28 | 35 |
| Uninoculated control | 0 | 0 | 0 | 0 |
| B.j. 110 control | 0.003 | 0.010 | 0.018 | 0.028 |
| 110 + 86-64 | 0.003 | 0.012* | 0.028* | 0.038* |
| 110 + G2-8 | 0.002 | 0.015* | 0.028* | 0.040* |
| 110 + G2-9 | 0.002 | 0.016* | 0.021* | 0.036* |
| 110 + G8-5 | 0.002 | 0.019* | 0.027* | 0.045* |
| 110 + G8-6A | 0.001 | 0.017* | 0.031* | 0.040* |
| 110 + G8-32 | 0.002 | 0.017* | 0.026* | 0.045* |
| 110 + G11-32 | 0.005 | 0.017* | 0.036* | 0.033 |

12

| 110 + G11-57 | 0.007* | 0.022* | 0.036* | 0.037 |
| LSD | 0.003 | 0.007 | 0.009 | 0.010 |

a          means of 5 plants/treatment.

*          Significantly different from the B. japonicum control (P=0.05).

## EXAMPLE 6 - EFFECTS OF NPR ON NITROGEN-FIXATION BY NODULATED SOYBEANS

The reduction of acetylene to ethylene is an indirect measurement of the potential ability of plants to fix nitrogen. In general terms, the value for ethylene evolved can be multiplied by three to determine how much nitrogen is actually being converted to ammonium ions (Hardy et al, 1973, Soil Biol. Biochem. 5:47-81).

For the acetylene reduction assay, soybean seeds were inoculated with appropriate B. japonicum and NPR treatments, planted in pots containing a field soil:perlite (1:1) mix and were grown in a greenhouse. Plants were sampled for nodulation and acetylene reduction data at 7, 14, 21, 28 and 35 days after planting.

The individual plants were carefully removed from the pots, and the soil was gently shaken from the roots. The plants were placed in a 1 litre Schott bottle (1 plant per bottle), and a silicone septum was placed on the mouth of the bottle, which was then tightly sealed with a screw cap. 100 ml of air was removed from each bottle and was replaced with 100 ml of acetylene, after which the bottles were incubated for one hour at room temperature. The concentration of acetylene reduced to ethylene by the plants was assayed with a gas chromatograph (Perkin Elmer 8310) equipped with a flame ionization detector and a 1000 x 3.2 mm Porapak N1 80/100 mesh column, by injecting 1-ml samples from the bottles. The operating conditions of the gas chromatograph were as follows: oven temperature, 85°C; detector temperature, 135°C; injection-port temperature, 90°C, and nitrogen was used as a carrier gas at flow rate of 20 ml/min. Ethylene standards were made of LOG 7 mole of pure gas (Wetmore welding Ltd., Toronto, Ontario, Canada M8Z 5K4). Five plants were assayed for each treatment. The treatment means were compared with the ANOVA and LSD tests.

The results are given in Table 7.

EP 0 227 336 B1

TABLE 7

| EFFECT OF NODULATION-PROMOTING RHIZOBACTERIA ON THE REDUCTION OF ACETYLENE TO ETHYLENE BY NODULATED SOYBEANS (nM $C_2H_4$/h/ROOT SYSTEM)[a] | | | | |
|---|---|---|---|---|
| Treatment | Age of Plants (Days) | | | |
| | 14 | 21 | 28 | 35 |
| Uninoculated control | 0 | 0 | 0 | 0 |
| B.j. 110 control | 1.5 | 70.3 | 89.6 | 180.5 |
| 110 + 86-64 | 5.8 | 121.2 | 122.3 | 180.9 |
| 110 + G2-8 | 3.0 | 158.4 | 183.9* | 323.1 |
| 110 + G2-9 | 6.9 | 136.4 | 132.4 | 445.9* |
| 110 + G8-5 | 7.3 | 98.2 | 146.3 | 522.4* |
| 110 + G8-6A | 5.1 | 188.7* | 148.4 | 522.8* |
| 110 + G8-32 | 12.1 | 208.4* | 130.1 | 488.3* |
| 110 + G11-32 | 27.5* | 291.5* | 187.7* | 187.0 |
| 110 + G11-57 | 25.8* | 382.1* | 150.5 | 460.9* |
| LSD | 20.3 | 106.9 | 69.9 | 146.4 |

a means of 5 plants/treatment.

* Significantly different from the B. japonicum control (P = 0.05).

As shown by the results reported in Table 7, all NPR strains tested increased the reduction of acetylene by nodulated soybeans. Of particular interest is that several tested NPR strains dramatically stimulated the rates of acetylene reduction, and hence increased nitrogen fixation ability, as soon as fourteen days after planting, indicating that co-inoculation with NPR may result in earlier nodule maturation, and thus earlier onset of nitrogen fixation. These results also indicate that nodules formed in the presence of NPR are capable of fixing greater amounts of nitrogen.

EXAMPLE 7 - EFFECTS OF NPR STRAINS ON NODULATION WHEN APPLIED TO SOYBEANS AS PEAT-BASED INOCULANTS

Two of the NPR strains previously identified and isolated, namely G2-8 ATCC 53288 and 86-64 ATCC 53289, were grown in TSB and harvested as previously described. The concentrated NPR suspensions were added to fine ground peat (90 ml of bacteria to 120 g of fine-ground peat) and were thoroughly mixed, using one individual strain per peat sample. The peat inoculants were "cured" by a two-week incubation at 30°C, to allow proliferation of the NPR within the peat medium.

B. japonicum 110 was prepared as a granular peat inoculant by growing the cells in YEM broth, followed by washing. A concentrated suspension was then mixed with the granular peat and was cured for two weeks at 30°C.

'Maple Arrow' soybean seeds were inoculated by mixing the seeds with NPR peat inoculant prepared as described above, and the seeds were planted in a field containing a clay-loam soil. The field was determined to be free from indigenous rhizobia.

The seeds were planted using a mechanical planter in the following fashion: A furrow was opened by the planter, granular peat containing B. japonicum 110 was drilled into the furrow, after which the NPR-inoculated seed was dropped on top of the granular peat. The furrow was then mechanically closed.

The plants were harvested after eight weeks, and the numbers of nodules per root system were counted and their dry weights determined. The results of this analysis are described in Table 8.

14

TABLE 8

| NODULATION OF MAPLE ARROW SOYBEANS WHEN INOCULATED WITH FINE-GROUND PEAT CONTAINING NPR AND PLANTED OVER GRANULAR PEAT CONTAINING B. JAPONICUM 110. | | | | |
|---|---|---|---|---|
| TREATMENT | NUMBER OF NODULES | % INCREASE OVER B.j.CONTROL | DRY WT.OF OF NODULES(g) | % INCREASE OVER B.j.CONTROL |
| B.j.110 control | 10.3 | | 0.052 | |
| 110 + G2-8 | 25.3 | + 146% | 0.084 | + 62% |
| 110 + 86-64 | 23.0 | + 123% | 0.102 | + 96% |

These results indicate that nodulation doubled 100% when soybean seeds were inoculated with fine-ground peat containing NPR and planted over granular peat containing B. japonicum 110.

EXAMPLE 8:   EFFECTS OF NPR ON NODULATION OF SOYBEANS GROWN IN SOIL CONTAINING INDIGENOUS B. JAPONICUM

Selected NPR strains from those previously reported were grown in TSB and harvested as previously described. Soybean seeds were inoculated with NPR by soaking them in the bacterial treatments (1 NPR strain per treatment at LOG 8 cfu/ml of inoculant suspension). The seeds were then planted in a field that had cropped soybeans the previous year and in which the presence of indigenous rhizobia had been confirmed.

The plants were harvested after eight and twelve weeks and the number of nodules per root system and nodule dry weights were determined. The results of these analyses are presented in Table 9.

TABLE 9

EFFECT OF NPR ON NODULATION OF SOYBEANS GROWN IN
FIELD SOIL CONTAINING INDIGENOUS B. JAPONICUM.

| TREATMENT | NUMBER OF NODULES | % INCREASE OVER B.j.CONTROL | DRY WT.OF OF NODULES(g) | % INCREASE OVER B.j.CONTROL |
|---|---|---|---|---|
| 8 weeks | | | | |
| Uninoculated control | 12.3 | | 0.117 | |
| 86-64 | 23.3 | + 89% | 0.150 | + 28% |
| G2-8 | 15.9 | + 29% | 0.141 | + 21% |
| G2-9 | 17.9 | + 46% | 0.150 | + 28% |
| G2-26 | 16.5 | + 34% | 0.148 | + 26% |
| G8-4 | 21.5 | + 75% | 0.213 | + 82% |
| G8-5 | 21.8 | + 77% | 0.220 | + 85% |
| G8-6A | 14.6 | + 19% | 0.180 | + 54% |
| 12 weeks | | | | |
| Uninoculated control | 18.9 | | 0.393 | |
| 86-64 | 39.4 | +108% | 0.920 | +134% |
| G2-8 | 28.3 | +50% | 0.576 | +47% |
| G2-9 | 24.6 | +30% | 0.507 | +29% |
| G2-26 | 29.9 | +58% | 0.632 | +61% |
| G8-4 | 35.7 | +89% | 0.965 | +146% |
| G8-5 | 41.1 | +117% | 0.819 | +108% |
| G8-6A | 27.5 | +46% | 0.501 | +27% |

These results indicate that NPR strains can substantially improve nodulation by indigenous strains of B. japonicum, and that this effect is maintained over extended periods of time.

EXAMPLE 9: GNOTOBIOTIC ASSESSMENT OF NODULATION PROMOTION BY NPR STRAINS

Preparation of Inoculum

Bradyrhizobium japonicum strain 61A101C was grown for 7 days in Glycerol Glutamate Yeast Extract Broth at 30°C (Glycerol 10.0g, Sodium Glutamate 1.0g, Yeast Extract 1.0g, $K_2HPO_4$ 0.5g, $KH_2PO_4$ 0.5g, $MgSO_4.7H_2O$ 0.2g, NaCl 0.2g, $FeSO_4$ 0.25g, per litre distilled water). Other bacterial strains were grown in Tryptic Soy Broth (Difco) at 30°C for 48 hrs. To prepare inoculants 1.5ml of B. japonicum culture and 1.0ml of other bacteria were added to 99ml of 0.1M $MgSO_4$.

Sterile tissue culture flasks (Corning 75cm$^2$) were filled with autoclaved 50:50 sand/vermiculite mixture (volume:volume to which 10g of $CaCO_3$ was incorporated per kg of sand). Sterile nutrient solution of Evans, Koch and Klucas, 1972 (Methods of Enzymology p 470-476, Vol. XXCIV Academic Press) was added to flasks filled with medium at 100ml/flask.

Soybean seeds (Glycine max L. Merr cultivar 'Maple Arrow') were surface sterilized in 1.5% sodium hypochlorite for 2 min. rinsed in sterile water and followed by immersion in 70% ethanol for 2 min., and rinsed in sterile distilled water. Seeds were planted in sterile moist vermiculite and incubated at 24°C for 40 hours in the dark to initiate germination.

Pre-germinated seeds were asceptically planted into the tissue culture flasks to a depth of 2cm, 1.0ml of the bacterial + B. japonicum solution was pipetted upon the seed and the seed was gently covered with moist sand/vermiculite. Eleven replicates per treatment were planted in this manner as were B. japonicum 61A101c alone and uninoculated controls (MgSO₄ solution alone). The plants were arranged in a randomized complete block design. Soybean plants were grown in a growth chamber for 28 days with a 14 hour photoperiod of approximately 200 u einsteins per sec. per cm², with a maximum and minimum temperature of 25° and 21°C respectively.

Following 28 days of growth, plants were harvested, nodules were excised, counted and dry weights of nodules determined. Statistical analysis was conducted by ANOVA and the Duncan's multiple range test.

As presented in Table 10 the greatest effect of bacterization is on nodule mass. All bacterization treatments increased nodule mass accumulation from 12-32% and 5 strains elicited a statistically significant response (P = .05). Statistically significant enhancement of nodule mass accumulation was observed by treatment with Serratia fonticola, S. proteamaculans, and Pseudomonas putida. Two strains (2-114 and 1-102) significantly enhanced nodule number. Based upon these observations it is assumed that bacterization treatments have their greatest effect on stimulating nodule development through enhancing rhizobia infectivity. This experiment also demonstrates that nodulation enhancement, via NPR strains such as G11-32, is not rhizobial strain specific, as nodulation enhancement has been observed with either USDA 110 or 61A101c as the Bradyrhizobium inoculum.


TABLE 10


INFLUENCE OF NPR STRAINS ON THE NODULATION OF MAPLE ARROW
SOYBEANS WITH 61A101C AS THE RHIZOBIAL INOCULANT
IN GNOTOBIOTIC ASSAY

| Strain# | Identification | Nodule mass mg/plant | Nodule number #/plant |
|---------|----------------|----------------------|-----------------------|
| 2-114 | Serratia fonticola | 89.2 A* | 65.8 A** |

| 1-102 | Serratia proteamaculans | 86.7 | AB | 63.0 | AB |
| G8-4 | Pseudomonas putida | 83.7 | AB | 60.7 | ABC |
| G2-8 | P. putida | 81.8 | AB | 60.1 | ABC |
| G11-32 | P. putida | 82.8 | AB | 56.7 | ABCD |
| G8-5 | P. fluorescens | 76.2 | ABC | 52.2 | BCD |
| G14-21 | P. putida | 72.1 | BC | 51.6 | CD |
| G2-26 | P. putida | 77.2 | ABC | 51.0 | CD |
| G12-22 | P. fluorescens | 76.5 | ABC | 50.1 | CD |
| 86-64 | Bacillus megaterium | 77.8 | ABC | 49.7 | CD |
| G8-32 | P. putida | 75.6 | ABC | 46.8 | D |
| | Bradyrhizobium japonicum control | 67.5 | C | 51.4 | CD |

\*   P   .05 level

\*\*  P   .10 level

It is apparent from Table 10 that dual inoculation by NPR plus strains of B. japonicum results in enhanced nodule number and nodule mass. Accordingly, the absence of Bradyrhizobium from soil (e.g. in sterile soil) can be compensated by the addition to the NPR inoculum of a suitable Bradyrhizobium strain to achieve enhanced nodule growth.

EXAMPLE 10: EFFECT OF NPR STRAINS WHEN APPLIED TO SOYBEAN SEED AS LYOPHYLIZED OIL FORMULATIONS IN FIELD TRIALS

Non-rhizobial NPR strains were grown in Tryptic Soy Broth (Difco) for 48 hours and harvested by centrifugation. Harvested cells were resuspended in 1/100 original volume of 2% mannitol, dispensed into vials, and quick frozen in ethanol dry ice for lyophilization. Lyophilized preparations of bacterial strains typically had a viability of Log 9 to Log 11 cells per gram.

Bradyrhizobium japonicum strain 122SR was grown in glycerol glutamate yeast extract broth for 7 days and blended with granular peat and calcium carbonate at a rate of 2015g of peat and 210g of calcium carbonate per litre of broth. Peat rhizobial cultures were allowed to incubate for 4 weeks at room temperature for regrowth and curing. Approximately $5 \times 10^8$ cells of B. japonicum were obtained per gram of peat prior to use.

Soybean seeds were inoculated by blending 540mg of lyopholized bacterial preparation with 8.0ml soybean oil per kg of soybean seed.

Soybean seeds were planted in three field trials containing a clay loam soil and a native population of soybean rhizobia, utilizing mechanical planting. Two trials were located near Milton, Ontario and one near Georgetown, Ontario.

The experiments were set up as randomized complete block designs with six replicates (except for the Georgetown location which had 8). The experimental design included a soybean oil control. All treatments received the granular B. japonicum inoculum at an application rate of 1.0g/metre of row.

Plants were harvested at the early reproductive stage of development (8-9 weeks after planting), and the dry weights of nodules for 10 plants per experimental plot were determined. The results of the analysis are described in Table 11. Statistical significance was determined by ANOVA, and Duncan's Multiple Range test was conducted.

In these three field studies Pseudomonas putida strain G11-32 elicited a statistically significant consistent enhancement of nodulation (nodule dry weight), compared to the oil control, of approximately 55%. Pseudomonas putida strain G2-26 elicited a consistent nodule mass enhancement in all 3 sites (39-59%) with statistical significance in 2 of 3 sites. Bacillus strain 86-64 elicited an enhancement of nodulation (41 and 60%) in both Milton experiments, and this enhancement was statistically significant in one of the experiments. These results indicate that coinoculation of soybean seeds with lyophilized cell preparations of

18

certain bacterial strains such as G11-32 can consistently elicit statistically significant enhancement of nodulation in soils containing an indigenous population of B. japonicum in replicated field trials. The enhancement of nodulation was observed in growth chamber and field studies, regardless of B. japonicum strain or location.

## TABLE 11

### NODULATIONS ENHANCEMENT OF SOYBEAN USING NODULATION PROMOTING RHIZOBACTERIA IN THREE ONTARIO FIELD EXPERIMENTS

| | Nodule Mass (gram dry weight/10 plants) | | | |
|---|---|---|---|---|
| | Site and Cultivar | | | Average Increase Compared to Control |
| Bacterial treatment | Georgetown 'Maple Arrow' | Milton 'Maple Arrow' | Milton 'Evans' | |
| 86-64 | 1.63 (-8%) | 1.54 (+41%) | 2.47* (+60%) | + 31% |
| G11-32 | 2.78*(+56%) | 1.66*(+52%) | 2.40* (+56%) | + 55% |
| G14-21 | 1.45 | 0.97 | 1.58 | |
| G8-32 | 1.36 | 1.82*(+67%) | 1.36 | |
| G2-26 | 2.66*(+49%) | 1.52 (+39%) | 2.46* (+60%) | + 49% |
| G12-22 | 1.07 | 0.65 | 1.40 | |
| G8-4 | 1.30 | 1.12 | 0.92 | |
| G8-5 | 1.70 | 0.85 | 2.00 | |
| G2-8 | 1.68 | 1.46 | 1.32 | |
| 1-102 | 1.45 | nt | nt | |
| 2-114 | 1.12 | nt | nt | |
| oil control | 1.78 | 1.09 | 1.54 | |

All plots received granular U.S.D.A. 122 B. japonicum inoculum.

\*      Significantly different than control at the 95% confidence level.

( )      % change from oil control.

## Claims

1.  A bacterial culture comprising at least one rhizobacterial strain that promotes nodulation of a leguminous plant and a Bradyrhizobium strain.

2.  A bacterial culture according to claim 1, wherein the rhizobacterial strain is selected from a strain of Bacillus megaterium; Pseudomonas putida; Serratia liquefaciens; Pseudomonas fluorescens; Serratia protoeamaculans ss. quinovora; and Serratia fonticola.

**3.** A bacterial culture according to claim 2, wherein the rhizobacterial strain is selected from Bacillus megaterium ATCC 53289; Pseudomonas putida ATCC 53288; Serratia proteamaculans ss. quinovora ATCC 53287; Pseudomonas putida ATCC 53286; Pseudomonas fluorescens ATCC 53449; Serratia fonticola ATCC 53450 and clones and subclones thereof.

**4.** A bacterial culture according to any one of claims 1 to 3, wherein said nodulation-promoting rhizobacterial strain is present as an axenic culture of said strain.

**5.** A process for enhancing the nodulation of leguminous plant roots by rhizobial bacteria, which comprises applying to the root-growth environment of the legume, an effective amount of a bacterial culture according to any one of claims 1 to 4.

**6.** A process according to claim 5, wherein the leguminous plant is soybean.

**7.** A process according to claim 6, wherein the Bradyrhizobium strain is Bradyrhizobium japonicum.

**8.** A process according to any one of claims 5 to 7, wherein the bacteria to be applied to the root-growth environment are applied by inoculating the legume seeds therewith, prior to planting.

**9.** A process according to any one of claims 5 to 8, wherein the bacterial culture is applied to said seed in an amount sufficient to establish a population of nodulation promoting rhizobacterial strain of from log 4 to log 6 cells per seed.

**10.** A process according to any one of claims 5 to 9, wherein the bacterial culture is applied to said seed in an amount sufficient to establish a population of the Bradyrhizobium strain of from log 4 to log 6 cells per seed.

**11.** An agricultural inoculant composition comprising (i) at least one rhizobacterial strain that promotes nodulation of leguminous plants, (ii) a Bradyrhizobium strain, (iii) an agriculturally acceptable carrier therefor.

**12.** An agricultural inoculant composition according to claim 11, wherein the Bradyrhizobium strain is Bradyrhizobium japonicum.

**13.** Legume seed having coated thereon a composition as defined in claim 11 or claim 12.

**14.** Soybean seed having coated thereon a composition as defined in claim 11 or claim 12.

**15.** A biologically pure culture which is designated herein as Serratia proteamaculans ss. quinovora 1-102 having ATCC accession number 53287, deposited on 10th October 1985, clones and sub-clones thereof.

**Patentansprüche**

**1.** Eine Bakterienkultur mit mindestens einem rhizobakteriellen Stamm, welcher die Nodulation von Hülsenfruchtpflanzen fördert und einem Bradyrhizobiumstamm.

**2.** Eine Bakterienkultur entsprechend Anspruch 1, wobei der rhizobakterielle Stamm aus den Stämmen Bacillus megaterium; Pseudomonas putida; Serratia liquefaciens; Pseudomonas fluorescens; Serratia proteamaculans ss. quinovora; und Serratia fonticola ausgewählt ist.

**3.** Eine Bakterienkultur gemäß Anspruch 2, wobei der rhizobakterielle Stamm aus Bacillus megaterium ATCC 53289; Pseudomonas putida ATCC 53288; Serratia proteamaculans ss. quinovora ATCC 53287; Pseudomonas putida ATCC 53286 Psuedomonas fluorescens ATCC 53449; Serratia fonticola ATCC 53450 und Klonen und Unterklonen hiervon ausgewählt ist.

**4.** Eine Bakterienkultur gemäß einem der Ansprüche 1-3, wobei der nodulationsfördernde rhizobakterielle Stamm in Form einer axenischen Kultur des vorgenannten Stammes vorliegt.

20

5. Ein Verfahren zur Förderung der Nodulation von Hülsenfruchtpflanzen-Wurzeln, durch rhizobiale Bakterien, dadurch gekennzeichnet, daß auf eine Wurzelwachstumsumgebung von Hülsenfrüchten eine effektive Menge einer Bakterienkultur entsprechend einem der Ansprüche 1 - 4 angewandt wird.

6. Ein Verfahren gemäß Anspruch 5, dadurch gekennzeichnet, daß die Hülsenfruchtpflanze eine Sojabohne ist.

7. Verfahren gemäß Anspruch 6, dadurch gekennzeichnet, daß als Bradyrhizobiumstamm Bradyrhizobium japonicum vorgesehen ist.

8. Ein Verfahren gemäß einem der Ansprüche 5-7, dadurch gekennzeichnet, daß die Bakterien, welche auf die Wurzelwachstumsumgebung angewandt werden, vor der Pflanzung durch Impfung der Hülsenfruchtsasten hiermit angewandt werden.

9. Ein Verfahren gemäß einem der Ansprüche 5-8, wobei die Bakterienkultur, welche auf die Saat angewandt wird, in einer Menge Anwendung findet, die ausreichend ist, um eine Population von nodulationsfördernden rhizobakteriellen Stämmen von log 4 - log 6 Zellen pro Saat zu etablieren.

10. Ein Verfahren gemäß einem der Ansprüche 5-9, wobei die Bakterienkultur auf die Saat in einer Menge angewandt wird, die genügend ist, eine Population des Bradyrhizobiumstamms von log 4 bis log 6 Zellen pro Saat zu etablieren.

11. Eine landwirtschaftliche Impfzusammensetzung mit:
   (i) mindestens einem rhizobakteriellen Stamm, welcher die Nodulation von Hülsenfruchtpflanzen fördert,
   (ii) einem Bradyrhizobiumstamm,
   (iii) einem landwirtschaftlich akzeptablen Träger hierfür.

12. Eine landwirtschaftliche Impfzusammensetzung entsprechend Anspruch 11, dadurch gekennzeichnet, daß als Bradyrhizobiumstamm das Bradyrhizobium japonicum vorgesehen ist.

13. Eine Hülsenfruchtsaat, bedeckt mit einer Zusammensetzung wie in den Ansprüchen 11 und 12 definiert.

14. Eine Sojabohnensaat, bedeckt mit einer Zusammensetzung wie in den Ansprüche 11 und 12 definiert.

15. Eine biologisch reine Kultur, welche hierin als Serratia proteamaculans ss. quinovora 1-102 bezeichnet ist, mit der ATCC Zugrifffsnummer 53287, hinterlegt am 10. Oktober 1985, deren Klone und Unterklone.

**Revendications**

1. Culture bactérienne comprenant au moins une souche rhizobactérienne favorisant la nodulation d'une légumineuse et une souche de Bradyrhizobium.

2. Culture bactérienne selon la revendication 1, dans laquelle la souche rhizobactérienne est sélectionnée parmi une souche de Bacillus megaterium ; Pseudomonas putida ; Serratia liquefaciens ; Pseudomonas fluorescens ; Setratia proteamaculans ss. quinovora; et Serratia fonticola.

3. Culture bactérienne selon la revendication 2, dans laquelle la souche rhizobactérienne est sélectionnée parmi Bacillus megaterium ATCC 53289 ; Pseudomonas putida ATCC 53288 ; Serratia proteamaculans ss. quinovora ATCC 53287 ; Pseudomonas putida ATCC 53286 ; Pseudomonas fluorescens ATCC 53449 ; Serratia fonticola ATCC 53450 et leurs clones et leurs sous-clones.

4. Culture bactérienne selon l'une quelconque des revendications 1 à 3, dans laquelle ladite souche rhizobactérienne favorisant la nodulation est présente sous la forme d'une culture axénique de ladite souche.

**5.** Procédé de stimulation de la nodulation de racines de légumineuses par des bactéries rhizobennes, lequel comprend l'application à l'environnement où se développent les racines de la légumineuse, d'une quantité efficace d'une culture bactérienne selon l'une quelconque des revendications 1 à 4.

**6.** Procédé selon la revendication 5, dans lequel la légumineuse est le soja.

**7.** Procédé selon la revendication 6, dans lequel la souche de Bradyrhizobium est Bradyrhizobium japonicum.

**8.** Procédé selon l'une quelconque des revendications 5 à 7, dans lequel les bactéries à appliquer à l'environnement où se développent les racines sont appliquées par inoculation aux semences de la légumineuse, avant leur plantation.

**9.** Procédé selon l'une quelconque des revendications 5 à 8, dans lequel la culture bactérienne est appliquée à ladite semence en une quantité suffisante pour établir une population de souche rhizobactérienne favorisant la nodulation comprise entre log 4 et log 6 cellules par semence.

**10.** Procédé selon l'une quelconque des revendications 5 à 9, dans lequel la culture bactérienne est appliquée à ladite semence en une quantité suffisante pour établir une population de la souche de Bradyrhizobium comprise entre log 4 et log 6 cellules par semence.

**11.** Composition d'inoculant agricole, comprenant (i) au moins une souche rhizobactérienne favorisant la nodulation de légumineuses, (ii) une souche de Bradyrhizobium, (iii) leur véhicule acceptable en agriculture.

**12.** Composition d'inoculant agricole selon la revendication 11, dans laquelle la souche de Bradyrhizobium est Bradyrhizobium japonicum.

**13.** Semence de légumineuse revêtue d'une composition telle que définie dans la revendication 11 ou la revendication 12.

**14.** Semence de soja revêtue d'une composition telle que définie dans la revendication 11 ou la revendication 12.

**15.** Culture biologiquement pure désignée ici comme étant Serratia proteamaculans ss. quinovora 1-102 portant le numéro ATCC 53287, déposée le 10 octobre 1985, ses clones et ses sous-clones.